Europäisches Patentamt

European Patent Office

Office européen des brevets

(19)

(11) Publication number: **0 399 736**
**A1**

## EUROPEAN PATENT APPLICATION

(21) Application number: 90305430.2

(22) Date of filing: 18.05.90

(51) Int. Cl.⁵: **A61M 5/14, F16K 7/06**

(30) Priority: 24.05.89 ES 891744

(43) Date of publication of application:
28.11.90 **Bulletin 90/48**

(84) Designated Contracting States:
**DE ES FR GB IT**

(71) Applicant: LEVENTON, S.A.
Poligono Can Sunyer, 11
E-08740 Santa Andreu da la Barca(ES)

(72) Inventor: Albanell, Armengol Jose
Poligono Can Sunyer 11
E-08740 Santa Andreu da la Barca(ES)
Inventor: Rodrigues, Sanchez
Poligono Can Sunyer 11
E-08740 Santa Andreu da la Barca(ES)

(74) Representative: Perry, Robert Edward et al
GILL JENNINGS & EVERY 53-64 Chancery
Lane
London WC2A 1HN(GB)

(54) **Flow control device.**

(57) A device for regulating the flow of liquid through a compressible tube (2), comprises:
a housing (1) carrying the tube;
a member (3) within the housing and in contact with the tube; and
adjustable means (4a) acting on the member (3) and thereby adapted to compress the tube adjustably; characterised in that the member has a configuration such the area thereof in contact with the tube varies according to the degree of compression of the tube.

*Fig. 1*

EP 0 399 736 A1

### Field of the Invention

This invention relates to a device for regulating the flow of a liquid through a compressible tube. Such devices have applications in therapy, since they are widely used in hospitals, to control the delivery of parenteral solutions administered by infusion.

### Background of the Invention

Many flow regulation devices are known, of varying degrees of sophistication. They are generally adapted to compress a flexible tube of plastics material and thereby regulate a flow rate of, say, 20-150 ml/h down to zero. Depending on the nature of the device, this control can be more or less accurate. More sophisticated devices are calibrated.

Three particular problems associated with known devices can be identified. One is that, especially for simple devices, it is difficult to change the flow rate by a small amount. Secondly, while many devices depend on the movement of a roller whose axle is constrained to move in a channel, it is easy to dislodge the roller from a given setting, directly or as the result of tension applied to the tubing.

For any given degree of compression of the flexible tube, defining a certain cross-sectional area through which liquid can flow, it is desirable that the flow rate should remain constant with time. The third problem is that suitable plastics tubing material, especially the widely-used PVC, tend to creep, on compression. With time, the tube undergoes cold flow. The consequence is that an initially constant flow rate drifts. This is particularly so in the case where the member which compresses the tube has, as is often the case, a rounded profile in contact with the tube.

Flow regulators are described in, for example, US-A-3099429, US-A-3289999, US-A-3298367, US-A-3299904, US-A-3477454, US-A-3477686, US-A-3497175, US-A-3584830, US-A-3612474, US-A-3625472, US-A-3685787, US-A-3802463, US-A-3805830, US-A-3877428, US-A-3900184, US-A-3960149, US-A-4034773, US-A-4328946, US-A-4335866, EP-A-0246110 and EP-A-0270499. In the case of most of these known devices, e.g. the simple roller clamp described in US-A-3099429, there is the problem of flow rate regularity. Although the object of that specification is to provide "drift-free" operation, the roller being positioned fixedly at any of various settings, the material of the tube will undergo cold creep at any one setting.

US-A-4328946 discloses a device for regulating the flow of liquid through a compressible tube, in which the compressible tube passes through a housing; a pivoted member is in contact with the tube and an adjustable screw acts on the member and is thereby adapted to compress the tube adjustably. "Cold flow" is acknowledged as a problem, and it is suggested that the tubing on which the member acts should be a segment of silicone rubber which is more resilient than PVC. It is stated that flow rate drifts of very low magnitude are achieved, i.e. about 5-10% over a wide range of flow rate settings.

US-A-3685787 adopts another solution to the problem of cold flow, by providing a constricted passage into which an adjustable roller forces the compressed tube. Flow rate may be adjusted by varying the ratio of the section of clamped tubing and the remaining, operative wall portion of the tubing, or by deforming the operative wall portion.

The flow control device disclosed in US-A-4335866 comprises first and second rollers, respectively for coarse and fine adjustment of the flow rate, acting on opposite ends of a member having an elongate surface in contact with the tubing. The member has a shaped configuration in contact with the rollers. The movement of one roller causes the member to pivot about the other roller, and thereby more or less into contact with the tubing.

### Summary of the Invention

A flow regulation device according to the present invention is of the general type in which a housing carries compressible tubing, there is a member within the housing and in contact with the tubing, and adjustable means acts on the member and is thereby adapted to compress the tube adjustably; the novel feature is that the member has a configuration such that the area thereof in contact with the tubing varies according to the degree of compression of the tube. Thus, the member does not always act on the same point. A consequence is good flow rate resolution.

A device of the invention can be constructed simply and economically. There is a minimum of moving parts. It is simple to use, and reliable.

### Description of the Invention

The invention will now be described by way of example only with reference to the accompanying drawings, in which Figures 1, 2 and (on an enlarged scale) 3a/3b are each cross-sectional views of three different embodiments of the invention, respectively; Figs. 3a and 3b show the tube respectively uncompressed and compressed.

Each of the drawings shows a housing 1 having an inlet and an outlet 2a and 2b between which is placed a compressible length of tubing 2. The inlet and outlet can be connected to conventional tubing of, say, PVC. The length of tubing in the housing can be of a more resilient material, e.g. silicone rubber.

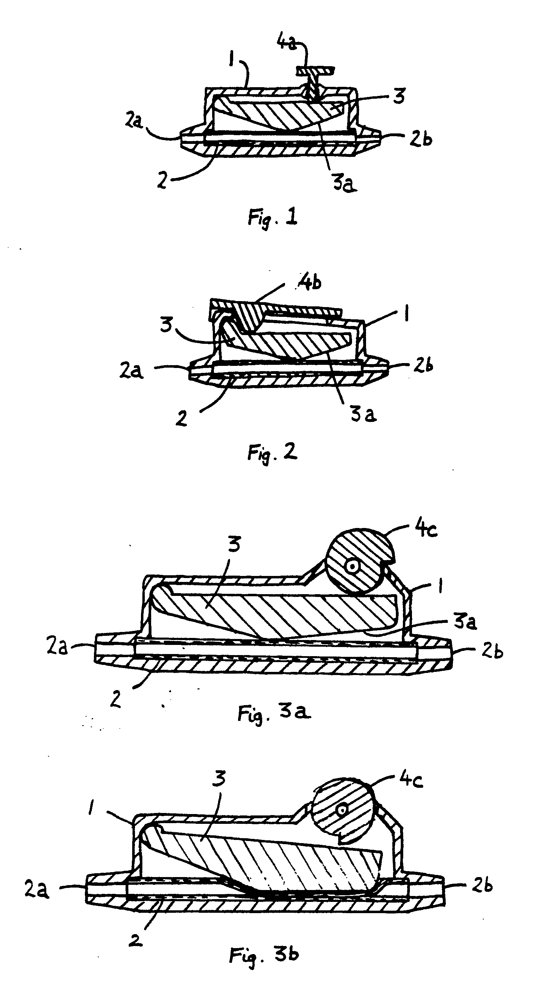

A member 3 is adapted to pivot about an axis of rotation defined by the bearing contact of the member on an internal angle of the housing 1. The member 3 has a lower (as illustrated) surface 3a adapted to come increasingly into contact with the tubing 2 as the member pivots under the influence of the respective adjustable means 4a, 4b and 4c illustrated in Figs. 1, 2 and 3.

Fig. 1 shows a screw member 4a. Depending on the pitch of the screw thread, a given number of turns of the screw 4a provides the difference between the "open" configuration shown in Fig. 1 and complete compression of the tubing 2.

Fig. 2 shows a member 4b slidably mounted in the housing 1. The "open" configuration is shown in Fig. 2; if the member 4b is slid to the right, the surface 3a of the member 3 is increasingly brought into contact with the tubing 2, until the tubing is closed.

A particularly preferred embodiment of the invention is shown in Figs. 3a and 3b, in which the adjustable means comprises an eccentric roller 4c mounted on a fixed axis. By rotating the roller 4c, e.g. by hand, through 270° anti-clockwise from the "open" position shown in Fig. 3a, the "closed" position shown in Fig. 3b is reached. Any intermediate position can also be maintained.

In a preferred device of the invention, the member is stably held within the housing by action/reaction at three points. One point is at the pivot. A second point is on one ("upper") surface of the member, where the adjustable means bears. The third point is on the opposite ("lower") surface, where the member is in contact with the tubing.

Strictly, the third point is a variable area. In a device of the invention, the ratio of the length of tubing affected by the member to the length over which the "lower" face of the member is applied (e.g. point contact up to, say, 20-50 mm in the closed position) is relatively low. Because the applied force is low, cold flow is reduced, in accordance with Hook's law.

By contrast with the device disclosed in US-A-4335866, the present invention requires neither two rollers nor that any roller is on a movable axis, nor is the configuration of the upper surface of the member critical. Indeed, it is the lower face which has a shaped configuration, which can be chosen according to the desired effect. The area which comes into contact with the tube will often be planar, but may be curved. It will usually be at an angle of 5-20° to the tube, in the open position, corresponding to the angle through which the member pivots through to the closed position. The member need not present a sharp angle of contact to the tube; it is, for example, 20-50°. This contrasts with the sharp contour of the shaped member in contact with the tubing in the device disclosed in US-A-4328946 or of the rollers disclosed in, say, US-A-3099429 or US-A-3685787.

## Claims

1. A device for regulating the flow of liquid through a compressible tube (2), which comprises: a housing (1) carrying the tube; a member (3) within the housing and in contact with the tube; and adjustable means (4) acting on the member (3) and thereby adapted to compress the tube adjustably; characterised in that the member (3) has a configuration such the area thereof in contact with the tube (2) varies according to the degree of compression of the tube (2).

2. A device according to claim 1, in which the member (3) is adapted to move about an axis of rotation defined by the bearing contact of the member (3) on an internal angle of the housing (1).

3. A device according to claim 1 or claim 2, in which the area of the member (3) in contact with the tube (2) is essentially planar.

4. A device according to any preceding claim, in which the adjustable means comprises an eccentric roller (4c) mounted on a fixed axis.

Fig. 1

Fig. 2

Fig. 3a

Fig. 3b

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-3 206 157 (READING)<br>* Complete document *<br>--- | 1 | A 61 M 5/14<br>F 16 K 7/06 |
| A,D | US-A-4 335 866 (BUJAN)<br>* Abstract; column 6, lines 8-37;<br>figures *<br>--- | 1,3,4 | |
| A | DE-A-2 341 309 (RUMP)<br>* Page 4, line 17 – page 5, line 20;<br>figures 1-6 *<br>--- | 1,2,3 | |
| A | AU-B- 492 047 (DERBY PRODUCTS PTY)<br>* Pages 4,5; figures *<br>----- | 1,2,3 | |
| | | | TECHNICAL FIELDS SEARCHED (Int. Cl.5)<br><br>A 61 M<br>F 16 K |

The present search report has been drawn up for all claims

| Place of search | Date of completion of the search | Examiner |
|---|---|---|
| THE HAGUE | 20-08-1990 | CLARKSON P.M. |